# EUROPEAN PATENT SPECIFICATION

(11) **EP 4 168 396 B1**
(45) Date of publication and mention of the grant of the patent: **31.07.2024**
(21) Application number: 21733476.2
(22) Date of filing: 23.06.2021
(51) Int. Cl.: C07D 401/06, C07D 401/14, A61P 25/16, A61P 25/28, A61K 31/513

(54) **COMPOUNDS FOR THE TREATMENT OF NEURODEGENERATIVE DISEASES**
VERBINDUNGEN ZUR BEHANDLUNG VON NEURODEGENERATIVEN ERKRANKUNGEN
COMPOSITION POUR LE TRAITEMENT DE MALADIES NEURODÉGÉNÉRATIVES

(30) Priority: 23.06.2020 EP 20305690
(43) Date of publication of application: 26.04.2023
(73) Proprietor: Universite De Franche Comte, 25030 Besançon (FR)
(72) Inventor: ISMAILI, Lhassane, 25000 Besançon (FR)
(74) Representative: Plasseraud IP
(86) International application number: PCT/EP2021/067252
(87) International publication number: WO 2021/260072

(56) References cited:
- JUSTYNA GODYN ET AL: "Therapeutic strategies for Alzheimer's disease in clinical trials", PHARMACOLOGICAL REPORTS, vol. 68, no. 1, 1 February 2016 (2016-02-01), PL, pages 127 - 138, XP055471351, ISSN: 1734-1140, DOI: 10.1016/j.pharep.2015.07.006
- CHOI DONG-YOUNG ET AL: "Natural products from marine organisms with neuroprotective activity in the experimental models of Alzheimer's disease, Parkinson's disease and ischemic brain stroke: their molecular targets and action me", ARCHIVES OF PHARMACAL RESEARCH, NATL. FISHERIES UNIVERSITY, PUSAN, KR, vol. 38, no. 2, 28 October 2014 (2014-10-28), pages 139 - 170, XP035442603, ISSN: 0253-6269, [retrieved on 20141028], DOI: 10.1007/S12272-014-0503-5

## Description

### BACKGROUND

In the central nervous system, memory and cognitive processes condition throughout our lives, our ability to learn and communicate as well as our psychomotor development. However, disturbances in memory and learning abilities can occur with age. Thus, with the increase in life expectancy worldwide, there is an alarming increase in the prevalence of neurodegenerative pathologies such as Alzheimer's disease (AD) in the elderly.

This disease leads to severe cognitive deficits such as disorientation, depression, and even inability to perform the tasks of daily living¹.

AD is characterized by a series of highly interconnected pathological processes that result, biologically and biochemically, in the accumulation and aggregation of an abnormal amount of extracellular deposits of amyloid-beta peptide (βA) that cause senile plaques and neurofibrillary degeneration, composed of hyperphosphorylated tau protein, leading to neuronal death². For the extracellular deposits (βA), several strategies have been developed to avoid these plaques and stop the progression of the disease, for example, using compounds that may act on the release of the peptide βA ^{3,4} or its aggregation ⁵.

Concerning the deposits related to neurofibrillary degeneration resulting from hyperphosphorylation of the tau protein, the main therapeutic strategies target the oligomerization of the protein and the inhibition this hyperphosphorylation^{6,7}, in particular the inhibition the Glycogen synthase kinase 3 (GSK-3β) which is the main kinase responsible for the phosphorylation of the Tau protein⁸.

Besides, over the last few decades, a cholinergic approach has brought 4 drugs to market for the treatment of the disease: Tacrine, Galanthamine, Rivastigmine and Donepezil. They are capable of inhibiting acetycholinesterase (AChE) and butyrylcholinesterase (BuChE), allowing an increase in neurotransmission between synapses to compensate for the cholinergic deficit by preventing hydrolysis of the neurotransmitter. ⁹

Another strategy for AD therapy is the development of drugs such as Nivaldipine, currently in phase 3 clinical development, able to block calcium channels ¹⁰. Indeed, the increase in the level of cytosolic calcium is involved in the physiopathology of AD, as it facilitates the formation of peptide βA by increasing the activity of calcium-induced beta secretase¹¹⁻¹⁵ and regulates certain kinases such as glycogen synthase kinase-3β (GSK-3β) responsible for the phosphorylation of the TAU protein and thus the formation of neurofibrilary deposits¹⁶. In addition, the entry of calcium through L-type calcium channels results in calcium overload and mitochondrial disruption leading to cell apoptosis activation¹⁷.

The Inventors have discovered that derived compounds from ethyl 1-(2-(1-benzylpiperidin-4-yl)ethyl)-6-methyl-2-oxo-4-(4-(3-(cycloamin-1-yl)alkoxy)phenyl)-1,2,3,4-tetrahydropyrimidine-5-carboxylate according to formula I, had an action on both calcium channel blockade, inhibition of cholinesterases (AChE and BuChE) and GSK-3β, inhibition of Tau aggregation.

In addition, they showed that these compounds also had an affinity for histamine H3 receptors, which are involved in the central regulation of histamine levels by retroinhibition of the release of histamine²¹, and other neurotransmitters such as acetylcholine, serotonin, dopamine and norepinephrine ²². The efficacy of H3R receptor antagonists in the treatment of AD has been demonstrated, for example through Contilisant ^{23,24}.

Thus, the compounds according to the invention have the advantage of targeting several causes of the disease.

### SUMMARY OF THE INVENTION

The present invention is directed to a compound of formula (I) 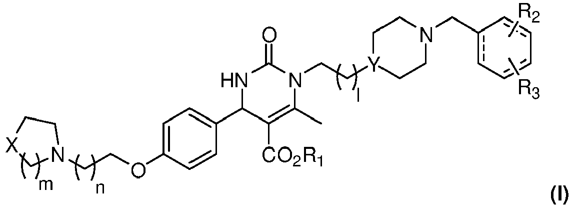 including any stereochemically isomeric form thereof, wherein :
Y is CH or N,
I is 0 or 1,
R₁ is a substituted or unsubstituted C₁-C₆ alkyl or a substituted or unsubstituted aryl,
R₂ and R₃ are independently of each other, are H, halogen, substituted or unsubstituted C₁-C₆ alkyl, cycloalkyl, substituted or unsubstituted aryl, NO₂, OH, CN, acyl, alkoxy, alkoxycarbonyl, SH,
X is O or CH₂,
m is 1 or 2,
n is 3, 4 or 5,
the symbol -̅ -̅ -̅ indicates a single or double bond;
or a pharmaceutically acceptable salt thereof.

The present invention is also directed to a pharmaceutical composition comprising a compound according to formula (I), or a pharmaceutically acceptable salt thereof, and a pharmaceutically acceptable carrier.

The present invention is further directed to a compound according to formula (I) or a pharmaceutically acceptable salt thereof, for use as a medicament, in particular for use in the treatment of neurodegenerative diseases.

### DETAILED DESCRIPTION

A compound according to the invention is inherently intended to comprise all stereochemically isomeric forms thereof. The term "stereochemically isomeric forms" as used hereinbefore or hereinafter defines all the possible stereoisomeric forms which the compounds of formula (I), may possess. Compounds of formula (I) can have one or more asymmetric carbon atoms and can exist in the form of optically pure enantiomers, mixtures of enantiomers such as, for example, racemates, optically pure diastereoisomers, mixtures of diastereoisomers, diastereoisomeric racemates or mixtures of diastereoisomeric racemates. The optically active forms can be obtained for example by resolution of the racemates, by asymmetric synthesis or asymmetric chromatography (chromatography with a chiral adsorbent or eluant). The invention embraces all of these forms. Unless otherwise mentioned or indicated, the chemical designation of compounds denotes the mixture of all possible stereochemically isomeric forms. In particular, stereogenic centers may have the D (+) or L (-) configuration; the R- or S-configuration; substituents on bivalent cyclic (partially) saturated radicals may have either the cis- or trans-configuration. Compounds encompassing double bonds can have an E (entgegen) or Z (zusammen)-stereochemistry at said double bond. The terms D, (+), L, (-), cis, trans, R, S, E and Z are well known to a person skilled in the art.

According to a first aspect, the present invention concerns a compound of formula (I): 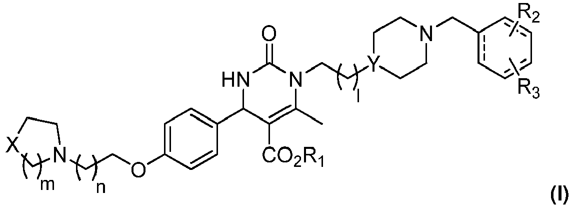 including any stereochemically isomeric form thereof, wherein:
Y is CH or N,
I is 0 or 1,
R₁ is a substituted or unsubstituted C₁-C₆ alkyl or a substituted or unsubstituted aryl,
R₂ and R₃ are independently of each other, are H, halogen, substituted or unsubstituted C₁-C₆ alkyl, cycloalkyl, substituted or unsubstituted aryl, NO₂, OH, CN, acyl, alkoxy, alkoxycarbonyl, SH,
X is O or CH₂,
m is 1 or 2,
n is 3, 4 or 5,
the symbol -̅ -̅ -̅ indicates a single or double bond
or a pharmaceutically acceptable salt thereof.

As used herein, the term "halogen" means Br, Cl, I and F.

The term "alkyl" as used herein denotes a substituted or unsubstituted chain, saturated, monovalent hydrocarbon residue containing 1 to 6 carbon atoms. "C₁-C₆ alkyl" as used herein refers to an alkyl composed of 1 to 6 carbons. Examples of alkyl groups include, but are not limited to, methyl, ethyl, propyl, isopropyl, butyl, sec-butyl, isobutyl, tert-butyl, pentyl and hexyl.

When substituted, there will generally be, for example, 1 to 4 substituents present on the alkyl. These substituents may optionally form a ring with the alkyl with which they are connected. Substituents may include, for example: carbon-containing groups such as alkyl, aryl, arylalkyl (e.g. substituted and unsubstituted phenyl, substituted and unsubstituted benzyl); halogen atoms and halogen-containing groups such as haloalkyl (e.g. trifluoromethyl); oxygen-containing groups such as alcohols (e.g. hydroxyl, hydroxyalkyl, aryl(hydroxyl)alkyl), ethers (e.g. alkoxy, aryloxy, alkoxyalkyl, aryloxyalkyl, more preferably, for example, methoxy and ethoxy), aldehydes (e.g. carboxaldehyde), ketones (e.g. alkylcarbonyl, alkylcarbonylalkyl, arylcarbonyl, arylalkylcarbonyl, arycarbonylalkyl), acids (e.g. carboxy, carboxyalkyl), acid derivatives such as esters (e.g. alkoxycarbonyl, alkoxycarbonylalkyl, alkylcarbonyloxy, alkylcarbonyloxyalkyl), amides (e.g. aminocarbonyl, mono- or di-alkylaminocarbonyl, aminocarbonylalkyl, mono- or di-alkylaminocarbonylalkyl, arylaminocarbonyl), carbamates (e.g. alkoxycarbonylamino, aryloxycarbonylamino, aminocarbonyloxy, mono- or di-alkylaminocarbonyloxy, arylminocarbonloxy) and ureas (e.g. mono- or di-alkylaminocarbonylamino or arylaminocarbonylamino); nitrogen-containing groups such as amines (e.g. amino, mono- or di-alkylamino, aminoalkyl, mono- or di-alkylaminoalkyl), azides, nitriles (e.g. cyano, cyanoalkyl), nitro; sulfur-containing groups such as thiols, thioethers, sulfoxides and sulfones (e.g. alkylthio, alkylsulfinyl, alkylsulfonyl, alkylthioalkyl, alkylsulfinylalkyl, alkylsulfonylalkyl, arylthio, arysulfinyl, arysulfonyl, arythioalkyl, arylsulfinylalkyl, arylsulfonylalkyl); and heterocyclic groups containing one or more heteroatoms, (e.g. thienyl, furanyl, pyrrolyl, imidazolyl, pyrazolyl, thiazolyl, isothiazolyl, oxazolyl, oxadiazolyl, thiadiazolyl, aziridinyl, azetidinyl, pyrrolidinyl, pyrrolinyl, imidazolidinyl, imidazolinyl, pyrazolidinyl, tetrahydrofuranyl, pyranyl, pyronyl, pyridyl, pyrazinyl, pyridazinyl, piperidyl, hexahydroazepinyl, piperazinyl, morpholinyl, thianaphthyl, benzofuranyl, isobenzofuranyl, indolyl, oxyindolyl, isoindolyl, indazolyl, indolinyl, 7-azaindolyl, benzopyranyl, coumarinyl, isocoumarinyl, quinolinyl, isoquinolinyl, naphthridinyl, cinnolinyl, quinazolinyl, pyridopyridyl, benzoxazinyl, quinoxalinyl, chromenyl, chromanyl, isochromanyl, phthalazinyl and carbolinyl).

As used herein, "substituted or unsubstituted aryl" means that said aryl may be unsubstituted or substituted by one or more substituents selected from the group consisting of alkyl, halogen, haloalkyl, alkoxy, haloalkoxy, alkylamino, NO₂, OH, CN, acyl, alkoxycarbonyl, SH.

The term "alkoxy" as used herein means an -O-alkyl group, wherein alkyl is as defined above.

According to the present application, "pharmaceutically acceptable" means that which is useful in preparing a pharmaceutical composition that is generally safe, non-toxic, and neither biologically nor otherwise undesirable and includes that which is acceptable for veterinary as well as human pharmaceutical use.

A "pharmaceutically acceptable salt" form of an active ingredient may also initially confer a desirable pharmacokinetic property on the active ingredient which were absent in the non-salt form, and may even positively affect the pharmacodynamics of the active ingredient with respect to its therapeutic activity in the body. The phrase "pharmaceutically acceptable salt" of a compound means a salt that is pharmaceutically acceptable and that possesses the desired pharmacological activity of the parent compound. Such salts include acid addition salts, formed with inorganic acids such as hydrochloric acid, hydrobromic acid, sulfuric acid, methanesulfonic acid, p-toluenesulfonic acid, carbonic acide, photonic acid, acetic acid, oxalic acid, succinic acid, tartaric acid, mandelic acid, fumaric acid, maleic acid, lactic acid, citric acid, glutamic acid, saccharic acid, monomethylimbic acid, 5-oxoproline acid, hexane-1-sulphonic acid, nicotinic acid, 2, 3 or 4-aminobenzoic acid, 2,4,6-trimethylbenzoic acid, lipoic acid, aspartic acid, hydrochloric acid. Other examples are mono- or bis-oxalates salt.

In a particular embodiment, the compound is a compound of formula (I) wherein Y is N. In a particular embodiment, the compound is a compound of formula (I) wherein Y is CH. In a particular embodiment, the compound is a compound of formula (I) wherein R₂ and R₃ are H. In a particular embodiment, the compound is a compound of formula (I) wherein R₁ is a C₁-C₆ alkyl such as an ethyl. In a particular embodiment, the compound is a compound of formula (I) wherein I is 0. In a particular embodiment, the compound is a compound of formula (I) wherein I is 1.

In a particular embodiment, the compound is a compound of formula (I) wherein Y is CH and R₂ and R₃ are H. In a particular embodiment, the compound is a compound of formula (I) wherein Y is CH and R₁ is a C₁-C₆ alkyl such as an ethyl. In a particular embodiment, the compound is a compound of formula (I) wherein R₂ and R₃ are H and R₁ is a C₁-C₆ alkyl such as an ethyl. In a particular embodiment, the compound is a compound of formula (I) wherein Y is CH, wherein R₂ and R₃ are H and wherein R₁ is a C₁-C₆ alkyl such as an ethyl.

In particular, the compound is a compound of formula (I) or a compound according to any one of the above particular embodiments, wherein X is CH₂. In particular, the compound is a compound of formula (I) or a compound according to any one of the above particular embodiments, wherein X is O. In particular, the compound is a compound of formula (I) or a compound according to any one of the above particular embodiments, wherein m is 1. In particular, the compound is a compound of formula (I) or a compound according to any one of the above particular embodiments, wherein m is 2. In particular, the compound is a compound of formula (I) or a compound according to any one of the above particular embodiments, wherein n is 3. In particular, the compound is a compound of formula (I) or a compound according to any one of the above particular embodiments, wherein n is 4. In particular, the compound is a compound of formula (I) or a compound according to any one of the above particular embodiments, wherein n is 5.In particular, the compound is a compound of formula (I) or a compound according to any one of the above particular embodiments, wherein X is CH₂ and m is 1. In particular, the compound is a compound of formula (I) or a compound according to any one of the above particular embodiments, wherein X is CH₂ and m is 2.

In particular, the compound is a compound of formula (I) or a compound according to any one of the above particular embodiments, wherein X is O and m is 1. In particular, the compound is a compound of formula (I) or a compound according to any one of the above particular embodiments, wherein X is O and m is 2.

In a particular embodiment, the compound is a compound of formula (II) corresponding to the formula (I) wherein Y is CH, R₂ and R₃ are H, R₁ is an ethyl and I is 1.

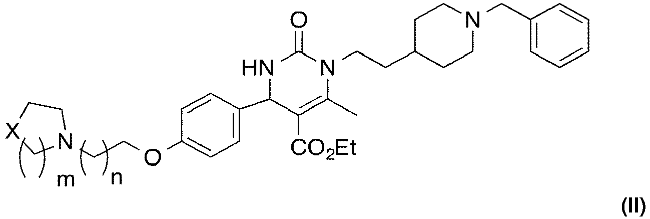

In a particular embodiment, the compound is a compound of formula (II) wherein X is CH₂. In a particular embodiment, the compound is a compound of formula (II) wherein X is O. In a particular embodiment, the compound is a compound of formula (II) wherein m is 1. In a particular embodiment, the compound is a compound of formula (II) wherein m is 2. In a particular embodiment, the compound is a compound of formula (II) wherein n is 3. In a particular embodiment, the compound is a compound of formula (II) wherein n is 4. In a particular embodiment, the compound is a compound of formula (II) wherein n is 5.

In a particular embodiment, the compound is a compound of formula (II) wherein X is CH₂ and m is 1. In a particular embodiment, the compound is a compound of formula (II) wherein X is CH₂ and m is 2.

In a particular embodiment, the compound is a compound of formula (II) wherein X is O and m is 1. In a particular embodiment, the compound is a compound of formula (II) wherein X is O and m is 2.

In a particular embodiment, the compound is a compound of formula (II) wherein X is CH₂, m is 1 and n is 3. The compound is then of the following formula:

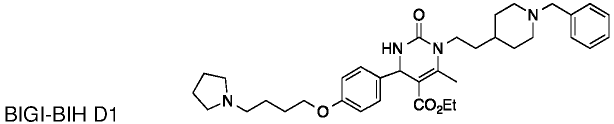

In a particular embodiment, the compound is a compound of formula (II) wherein X is CH₂, m is 2 and n is 3. The compound is then of the following formula:

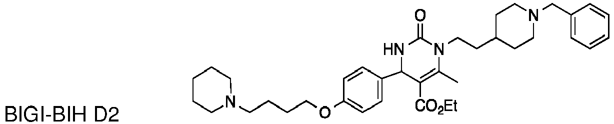

In a particular embodiment, the compound is a compound of formula (II) wherein X is O, m is 2 and n is 3. The compound is then of the following formula:

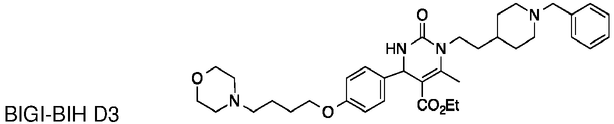

In a particular embodiment, the compound is a compound of formula (II) wherein X is CH₂, m is 1 and n is 4. The compound is then of the following formula:

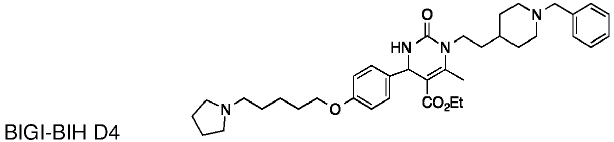

In a particular embodiment, the compound is a compound of formula (II) wherein X is CH₂, m is 2 and n is 4. The compound is then of the following formula:

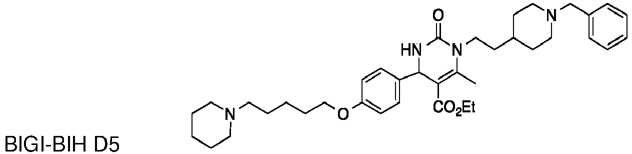

In a particular embodiment, the compound is a compound of formula (II) wherein X is O, m is 2 and n is 4. The compound is then of the following formula:

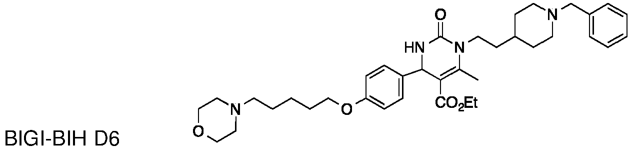

In a particular embodiment, the compound is a compound of formula (II) wherein X is CH₂, m is 1 and n is 5. The compound is then of the following formula:

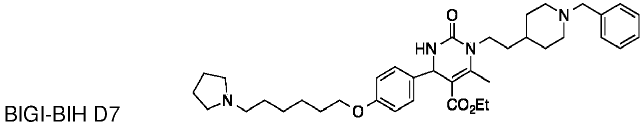

In a particular embodiment, the compound is a compound of formula (II) wherein X is CH₂, m is 2 and n is 5. The compound is then of the following formula:

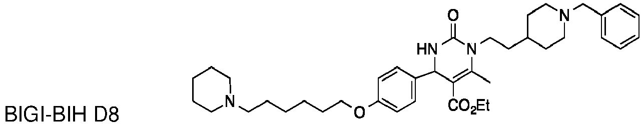

In a particular embodiment, the compound is a compound of formula (II) wherein X is O, m is 2 and n is 5. The compound is then of the following formula:

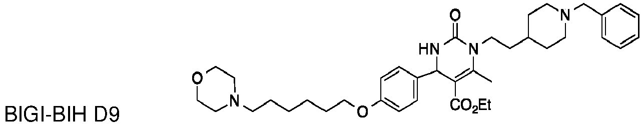

The present application also relates to a process for manufacturing compound of formula (II) wherein a Biginelli reaction is performed between ethyl acetoacetate, an urea of formula (A) (1-(2-(1-benzylpiperidin-4-yl)ethyl)urea) 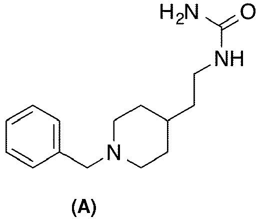 and an aldehyde of formula (III) 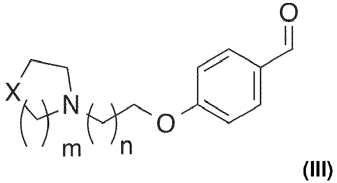 wherein X is O or CH₂, m is 1 or 2, n is 3, 4 or 5,

In particular, said Biginelli reaction is performed with sodium bisulfate (NaHSO₄) as catalyst, and more particularly in presence of acetic acid.

In a particular embodiment, the process for manufacturing compound of formula (II) comprises:
- a first reaction step between an aldehyde of formula (III) and 1-(2-(1-benzylpiperidin-4-yl)ethyl)urea of formula (A), followed by
- a second reaction step wherein ethyl acetoacetate is added, in particular with sodium bisulfate (NaHSO4).

In particular, the first reaction step lasts 2h. Typically, the second reaction step lasts 48h at room temperature (TA).

The present invention is also directed to a pharmaceutical composition comprising a compound as described above or a pharmaceutically acceptable salt thereof, and a pharmaceutically acceptable carrier.

The term "carrier" refers to a compound that is useful in preparing a pharmaceutical composition, generally safe, non-toxic and neither biologically nor otherwise undesirable, and includes excipients that are acceptable for human pharmaceutical uses. It encompasses carriers, excipients, and diluents and means a material, composition or vehicle, such as a liquid or solid filler, diluent, excipient, solvent or encapsulating material, involved in carrying or transporting a pharmaceutical agent from one organ, or portion of the body, to another organ, or portion of the body.

Useful pharmaceutical carriers for the preparation of the compositions hereof, can be solids, liquids or gases. The carrier can be selected from the various oils including those of petroleum, animal, vegetable or synthetic origin, e.g., peanut oil, soybean oil, mineral oil, sesame oil, and the like. Water, saline, aqueous dextrose, and glycols are representative liquid carriers, particularly (when isotonic with the blood) for injectable solutions. For example, formulations for intravenous administration comprise sterile aqueous solutions of the active ingredient(s) which are prepared by dissolving solid active ingredient(s) in water to produce an aqueous solution, and rendering the solution sterile.

Suitable pharmaceutical carriers include starch, cellulose, talc, glucose, lactose, talc, tragacanth, gelatin, pectin, dextrin, malt, rice, flour, chalk, silica, magnesium stearate, sodium stearate, glycerol monostearate, sodium chloride, methylcellulose, sodium carboxymethylcellulose, dried skim milk, glycerol, propylene glycol, water, ethanol, and the like. The pharmaceutical composition may be subjected to conventional pharmaceutical additives such as preservatives, stabilizing agents, wetting or emulsifying agents, salts for adjusting osmotic pressure, buffers, colorants, flavors, artificial and natural sweeteners, dispersants, thickeners, solubilizing agents and the like.

The compounds or compositions of the invention can thus be administered, for example, ocularly, orally (e.g., buccal cavity), sublingually, parenterally (e.g., intramuscularly, intravenously, or subcutaneously), rectally (e.g., by suppositories or washings), transdermally (e.g., skin electroporation) or by inhalation (e.g., by aerosol).

The composition of the invention may have a form of solid (e. g. include powders, tablets, coated tablets, dragees, pills, capsules, cachets, suppositories, and dispersible granules), liquid (e. g. solutions, emulsions, syrups, elixirs, or suspensions), gas or aerosol.

The present invention also relates to a compound as described above or a pharmaceutically acceptable salt thereof, or a pharmaceutical composition comprising said compound or a pharmaceutically acceptable salt thereof as described above, for use as a medicament.

In particular, it relates to a compound as described above or a pharmaceutically acceptable salt thereof, or a pharmaceutical composition comprising said compound or a pharmaceutically acceptable salt thereof as described above, for use in the treatment of a neurodegenerative disease, a central nervous system disease, narcolepsy or sleep disorder.

In particular, the neurodegenerative disease is selected in the group consisting of: Alzheimer's disease, Parkinson's disease, amyotrophic lateral sclerosis.

The present invention also concerns the use of a compound as described above or a pharmaceutically acceptable salt thereof, in the manufacture of a medicament for treating a neurodegenerative disease, a central nervous system disease, narcolepsy or sleep disorder.

According to the present disclosure, the term "treating", with regard to a subject, refers to improving at least one symptom of the subject's disorder. Treating can be curing, improving, or at least partially ameliorating the disorder.

### FIGURES

**Figure 1****:** Scheme of the synthesis of urea compound of formula (A) (1-(2-(1-benzylpiperidin-4-yl)ethyl)urea)
**Figure 2****:** Scheme of the synthesis of aldehydes of Formula (III), in particular aldehydes 2a-2i
**Figure 3****:** Scheme of the synthesis of the derivate compounds of ethyl 1-(2-(1-benzylpiperidin-4-yl)ethyl)-6-methyl-2-oxo-4-(4-(3-(cycloamin-1-yl)alkoxy)phenyl)-1,2,3,4-tetrahydropyrimidine-5-carboxylate of Formula (II), in particular the compounds BIGI-BIH D1-D9.

### MATERIAL & METHODS

### 1. Synthesis of the derivate compounds

### 1.1. Materials

All reagents were purchased from Sigma Aldrich or TCI. 1H and ¹³C NMR spectra were recorded on a Bruker spectrometer; operating at (1H NMR at 400 MHz, ¹³C NMR at 100 MHz), in solution in dimethylsulfoxide (DMSO-d₆) at 20 °C; chemical shift values are given in δ (ppm) relatively to TMS as internal reference. Coupling constants are given in Hz. The following abbreviations were used: s = singlet, d = doublet, t = triplet, q = quartet, m = multiplet. High resolution mass spectra were obtained at Centre Commun de Spectrométrie de Masse, Lyon, France on a Bruker micrOTOF-Q II spectrometer (Bruker Daltonics) in positive ESI-TOF (electrospray ionization-time of flight). The TFAHs were found to be ≥ 95% pure by HPLC analysis using a Hitachi Lachrom Elite series instrument equipped with a L2400 Lachrom Elite DAD detector and a Uptisphere ODB column (4.6 mm × 100 mm, Ø= 3 µm). Peaks were detected at 210 nm and the system was operated at 25 °C with a flow rate of 2 mL/min. The mobile phase was an isocratic mixture of acetonitrile and water (1:1, v/v) containing 0.1% (w/v) monopotassium phosphate.

### 1.2. General scheme of the synthesis

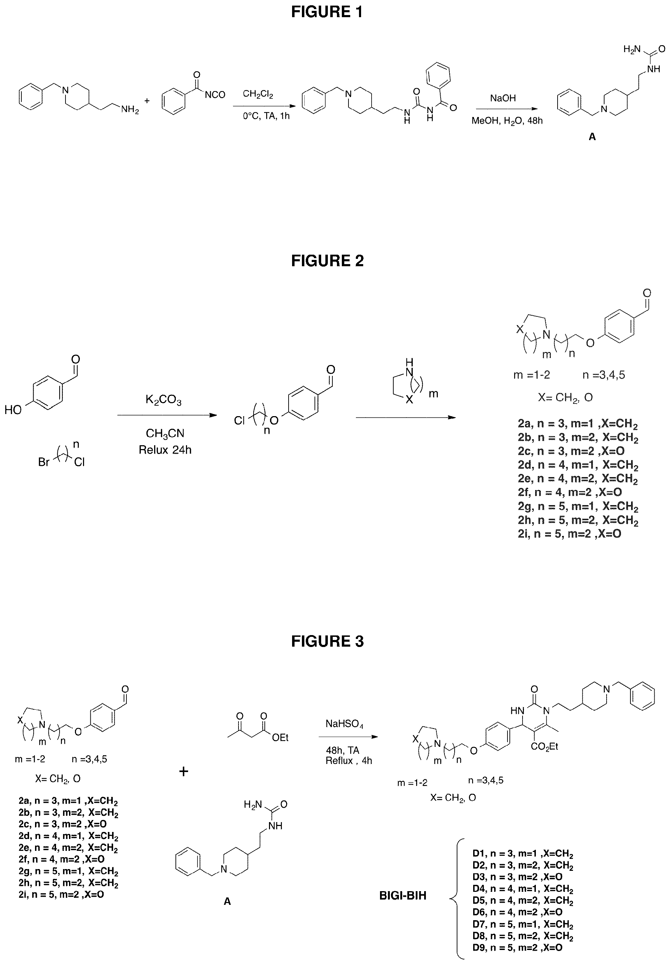

The synthesis of derivate compounds of ethyl 1-(2-(1-benzylpiperidin-4-yl)ethyl)-6-methyl-2-oxo-4-(4-(3-(cycloamin-1-yl)alkoxy)phenyl)-1,2,3,4-tetrahydropyrimidine-5-carboxylate (BIGI-BIH) has been carried out by a straightforward three-step synthesis with good overall yields, as depicted in Figures 1 to 3.

On a side, the reaction of commercial benzylpiperidine with the benzoyl isocyanate in CH₂Cl₂ at 0°C for 1 hour followed by hydrolysis of obtained compound with NaOH for 48h gave the expected urea compound A (1-(2-(1-benzylpiperidin-4-yl)ethyl)urea), as depicted in Figure 1. On another side, aldehydes compounds were prepared from 4-hydroxybenzaldehyde by alkylation with appropriate bromo-chloroalkane following by reaction with pyrrolidine, piperidine or morpholine as depicted in Figure 2. Next, the one-pot Biginelli reaction of urea compound A with aldehydes 2a-i and ethyl acetoacetate in acetic acid with sodium bisulfate as catalyst, gave the compounds BIGI BIH D1-D9 (Figure 3).

The aldehydes a-j and the compounds BIGI BIH D1-D9 have been characterized by NMR.

### 1.3. Synthesis of aldehydes 2a-i

A suspension of the corresponding benzaldehyde (1 equiv, 8.2 mmol), bromochloroalcane (1.5 equiv, 12.3 mmol) and potassium carbonate (3 equiv, 24.6 mmol) in acetonitrile (24 ml) was refluxed for 24 h. It was then filtered over celite and the filtrate evaporated. The residue was diluted with pentane and filtered over silica using first pentane as eluent then dichloromethane. The fraction obtained with dichloromethane was evaporated and the residue solubilised in acetonitrile (30 ml). Potassium carbonate (3 equiv, 24.6 mmol) and the appropriate amine (2 equiv, 16.4 mmol) were added and the mixture was stirred at reflux for 24 h. The suspension was filtered over Celite and the filtrate evaporated. The residue was dissolved in dichloromethane washed with water, brine, dried over anhydrous sodium sulfate and concentrated. It was then purified by Flash column chromatography, using CH₂Cl₂/MeOH/NH₃ (90 : 9.9 : 0.1) to afford the desired aldehyde.

### 1.4. Synthesis of compounds BIGI-BIH D1-D9 by Biginelli reaction using aldehydes 2a-i

The appropriate aldehyde (1 eq) and 1-(2-(1-benzylpiperidin-4-yl)ethyl)urea (1,2 eq) are solubilised in glacial acetic acid and the resulting mixture is stirred at room temperature for 2h. Ethyl acetoacetate (1.2 eq) and sodium bisulfate (1 eq) are added and the suspension left under stirring for 48h at room temperature, then refluxed for 4h. The catalyst is filtered. The filtrate is evaporated and purified by flash column chromatography using a mixture of CH2Cl2/MeOH/NH3 (90 : 9.9 : 0.1) to get the desired hybrid.

### 2. Biological activities

### 2.1. H3 Receptor Binding Assay

10 mM stock solutions of compounds to be tested were prepared in DMSO. Serial dilutions of compounds were prepared in 96-well microplate in assay buffers using automated pipetting system epMotion 5070 (Eppendorf Each compound was tested in 8 concentrations from 10-5 to 10-12 M (final concentration).

Radioligand binding was performed using membranes from CHO-K1 cells stably transfected with the human H3 receptor. All assays were carried out in duplicates. 50 µl working solution of the tested compounds, 50 µl [3H]-N-α-methylhistamine (spec. act. 82.9 Ci/mmol, final concentration 1.0 nM) and 150 µl diluted membranes (15 µg protein per well) prepared in assay buffer (50 mM Tris, pH 7.4, 5 mM MgCl2) were transferred to polypropylene 96-well microplate using 96-wells pipetting station Rainin Liquidator (MettlerToledo). (R)(-)-α-methylhistamine (100 µM) was used to define nonspecific binding. Microplate was covered with a sealing tape, mixed and incubated for 60 minutes at 27°C. The reaction was terminated by rapid filtration through GF/B filter mate presoaked with 0.5% polyethyleneimine for 30 minutes. Five rapid washes with 300 µl 50 mM Tris buffer (4°C, pH 7.4) were performed using 96-well FilterMate harvester (PerkinElmer, USA). The filter mates were dried at 37°C in forced air fan incubator and then solid scintillator MeltiLex was melted on filter mates at 90°C for 4 minutes. Radioactivity was counted in MicroBeta2 scintillation counter (PerkinElmer). Data were fitted to a one-site curve-fitting equation with Prism 5 (GraphPad Software) and Ki values were estimated from the Cheng-Prusoff equation.

### 2.2. Cholinesterase inhibition assay

Inhibitory activities of the test compounds against the human cholinesterases were measured using the spectrophotometric method described by Ellman et al.⁴, as modified for 96-well microplates. 5,5'-Dithiobis-(2-nitrobenzoic acid) (DTNB), acetylthiocholine iodide (ATC), butyrylthiocholine iodide (BTC), and human recombinant acetylcholinesterase (hAChE) were purchased from Sigma-Aldrich. Human butyrylcholinesterase (hBuChE) isolated from human plasma was from Vivonics (Bedford, MA, USA). The enzymes were prepared by dissolving in demineralized water to give stock solutions of 5 U/mL. hAChE and hBuChE were further diluted before use to a final concentration of 0.384 U/mL. The stock solutions of the test compounds were prepared by dissolving in DMSO to obtain a concentration of 0.0034M. They were further diluted in water to give appropriate concentration in a well. In the first step of Ellman's method 25 µL of the test compound (or mixture DMSO/water in appropriate ratio; i.e. blank samples) was incubated in 0.1 M phosphate buffer (200 µL, pH= 8.0) with DTNB (20 µL; 0.0025M) and the enzyme (20 µL; hAChE or hBuChE) in temperature 36 °C. Then after an incubation period of 5 min., 20 µL of ATC (0.00375M) or BTC (0.00375M) solutions (depending on the enzyme used) were added. After a further 5 min, the changes in absorbance were measured at 412 nm, using a microplate reader (EnSpire Multimode; PerkinElmer). All compounds were tested at screening concentration of 10 µM. Percent of inhibition was calculated from 100-(S/B)×100, where S and B were the respective enzyme activities with and without the test sample, respectively. For compounds with better than 50% inhibitory activity at 10 µM), IC50 values were determined. To determine IC50 value, six different concentrations of each compound were used to obtain enzyme activities between 5% and 95%. All reactions were performed in triplicate. The IC50 values were calculated using nonlinear regression (GraphPad Prism 5 [GraphPad Software, San Diego California USA 5.00]) by plotting the residual enzyme activities against the applied inhibitor concentration. Tacrine was used as a reference compound.

### 2.3. Calcium channel antagonist assay

The cytosolic Ca2+ has been measured in SH-SY5Y cells. SH-SY5Y cells were maintained similarly to what was previously described56. Free cytosolic Ca2+ was measured using the fluorescent Ca2+ indicator Fluo-4/AM (Fluo-4 acetomethoxyester). SH-SY5Y cells were seeded onto 96-well black plates at a density of 105 cells per well, achieving confluence after 48 h. Cells were washed with Krebs-Hepes solution (KH, in mM: 144 NaCl, 5.9 KCl, 1.2 MgCl2, 2 CaCl2, 11 D-glucose, 10 HEPES, pH 7.4). Cells were loaded with 10 µM Fluo-4/AM and 0.2% pluronic acid in KH and incubated for 45 min at 37 ºC in the dark. Then, cells were washed twice with KH to remove the excess of probe. Tested compounds were incubated 10 min before K+ 70 mM was applied to evoke the increment of cytosolic Ca2+. To normalize Fluo-4 signals, Triton X-100 (5%) and 1 M MnCl2 were applied to register both maximal and minimal fluorescence, respectively. The experiments were analyzed at excitation and emission wavelengths of 485 and 520 nm, respectively, and fluorescence was measured in a fluorescence microplate reader (FLUOstar Optima, BMG, Germany). Data were calculated as a percentage of Fmax - Fmin.

### 2.4. GSK-3β inhibition in vitro assay

Human recombinant GSK-3β, prephosphorylated polypeptide substrate and white 96-well plates were purchased from Millipore (Millipore Iberica S.A.U.). Kinase-Glo Luminescent Kinase Assay was obtained from Promega (Promega Biotech Iberica, SL). Adenosinetriphosphate (ATP) and all other reagents were from Sigma-Aldrich. Assay buffer contained 50 mM 4-(2-hydroxyethyl)-1- piperazineethanesulfonic acid (HEPES) (pH 7.5), 1 mM ethylenediaminetetraacetic acid (EDTA), 1 mM ethylene glycol tetraacetic acid (EGTA), and 15 mM magnesium acetate. The in solution experiments for GSK-3β inhibition analysis were performed by following the method developed by Baki et al.¹⁸. In a typical assay, 10 µL of test compound (dissolved in DMSO at 1 mM concentration and diluted in advance in assay buffer to the desired concentration) and 10 µL (20 ng) of enzyme were added to each well in presence of 20 µL of assay buffer containing GSM substrate and ATP in order to reach a 25 µM and 1 µM final concentration respectively. The final DMSO percentage in the reaction mixture did not exceed 5%. After a 30 min incubation time at 30 °C, the enzymatic reaction was stopped by adding 40 µL of Kinase-Glo reagent. Glow-type luminescence was recorded after 10 min using a VictorX 3 PerkinElmer plate reader. The activity was found proportional to the difference of the total and consumed ATP. The inhibitory activities were calculated on the basis of maximal kinase (average positive) and luciferase activities (average negative) measured in the absence of inhibitor and in the presence of reference compound inhibitor (SB-415286 Merck Millipore, IC₅₀ = 55 nM) at total inhibition concentration (5 µM)¹⁹, respectively. The linear regression parameters were determined and the IC₅₀ extrapolated (GraphPad Prism 4.0, GraphPad Software Inc.).

### 2.5. Oligomerization Tau protein inhibition

### Tau(306-336) pretreatment

1 mg of Tau(306-336) (Bachem AG, Germany) was initially dissolved in 1,1,1,3,3,3,-hexafluoro-2-propanol (HFIP), gently vortexed, sonicated, and kept overnight at room temperature. Subsequently, the sample was aliquoted dried and stored at -20 °C.

### Tau(306-336) peptide aggregation and its inhibition by ThT fluorimetric assay

Stock solutions of Tau(306-336) peptide (500 µM) were prepared in ultrapure water and immediately used. Stock solution of thioflavin T (ThT, 500 µM) was prepared in 56.3 mM phosphate buffer (PB, pH = 7.4) while stock solution of inhibitors (20 mM) were prepared in DMSO/methanol 10/90. Tau(306-336) aggregation was monitored at 30 °C in black, clear bottom 96-well plate (Greiner) by EnSpire multi-plate reader (Perkin Elmer) using ThT fluorimetric assay²⁰ with some variations. The excitation and emission wavelengths were set at 446 and 490 nm, respectively. Assay samples were prepared by diluting Tau(306-336) stock solution to 50 µM in the assay mixture which consisted in 20 µM ThT, 48.1 mM PB (final concentrations) in final 100 µL volume (final DMSO and MeOH content: 0.05% and 0.45%, respectively). Inhibition experiments were performed by incubating Tau(306-336) peptide at the given conditions in the presence of tested inhibitors at 50 µM. Fluorescence data were recorded every 10 min overnight with 1 min shaking at 800 rpm prior to each reading. Each inhibitor was assayed in triplicates in at least two independent experiments. Estimation of the inhibitory potency (%) was carried out by comparing fluorescence values at the plateau (average fluorescence intensity value in the 12 - 16 h range). Inhibition % are expressed as the mean ± SEM. Quenching of ThT fluorescence was evaluated by preparing blank solutions containing inhibitor/reference compound and preformed fibrils of Tau(306-336) peptide. Values were averaged and the presented values are expressed as the mean ± SEM.

### RESULTS

### 1. Synthesis of the derivate compounds: Examples of compounds BIGI-BIH D1-D9

Aldehydes 2a-i were prepared from 4-hydroxybenzaldehyde by alkylation with appropriate bromo-chloroalkane following by reaction with pyrrolidine, piperidine or morpholine (Figure 2).

The synthesis of the derivate compounds of ethyl 1-(2-(1-benzylpiperidin-4-yl)ethyl)-6-methyl-2-oxo-4-(4-oxo-4H-chromen-3-yl)-1,2,3,4-tetrahydropyrimidine-5-carboxylate has been carried out by a Biginelli reaction of corresponding aldehydes 2a-i, ethyl acetoacetate and compound A (urea) in acetic acid with sodium bisulfate as catalyst (Figure 3).

The NMR characterizations of aldehydes 2a-i and the derivate compounds BIGI-BIH D1 to BIGI-BIH D9 obtained by this process, are described below.

### Aldehyde 2a : 4-(4-(pyrrolidin-1-yl)butoxy)benzaldehyde

Yield: 21%.

¹H NMR (400 MHz, CDCl₃) δ 9.88 (s, 1H), 7.82 (d, *J* = 8.8 Hz, 2H), 6.98 (d, *J* = 8.7 Hz, 2H), 4.07 (t, *J* = 6.3 Hz, 2H), 2.67 - 2.54 (m, 6H), 1.89 - 1.73 (m, 8H).

¹³C NMR (101 MHz, CDCl₃) δ 190.82, 164.00, 132.01, 129.91, 114.75, 67.84, 55.51, 53.64, 26.95, 24.34, 23.38.

### Aldehyde 2b : 4-(4-(piperidin-1-yl)butoxy)benzaldehyde

Yield: 25%.

¹H NMR (400 MHz, CDCl₃) δ 9.88 (s, 1H), 7.82 (d, *J* = 8.8 Hz, 2H), 6.99 (d, *J* = 8.7 Hz, 2H), 4.07 (t, *J* = 6.4 Hz, 2H), 2.44 - 2.31 (m, 6H), 1.88 - 1.78 (m, 2H), 1.74 - 1.63 (m, 2H), 1.62 - 1.54 (m, 4H), 1.48 - 1.39 (m, 2H).

¹³C NMR (101 MHz, CDCl₃) δ 190.83, 164.20, 132.00, 129.80, 114.77, 68.23, 58.98, 54.65, 27.24, 26.03, 24.50, 23.44.

### Aldehyde 2c : 4-(4-morpholinobutoxy)benzaldehyde

Yield: 49%.

¹H NMR (400 MHz, CDCl₃) δ 9.87 (s, 1H), 7.82 (d, *J* = 8.8 Hz, 2H), 6.98 (d, *J* = 8.7 Hz, 2H), 4.07 (t, *J* = 6.3 Hz, 2H), 3.74 - 3.67 (m, 4H), 2.48 - 2.35 (m, 6H), 1.90 - 1.80 (m, 2H), 1.73 - 1.63 (m, 2H).

¹³C NMR (101 MHz, CDCl₃) δ 190.80, 164.11, 132.00, 129.85, 114.73, 68.09, 67.01, 58.51, 53.74, 27.00, 23.02.

### Aldehyde 2d : 4-((5-(pyrrolidin-1-yl)pentyl)oxy)benzaldehyde

Yield: 28%.

¹H NMR (400 MHz, CDCl₃) δ 9.88 (s, 1H), 7.83 (d, *J* = 8.8 Hz, 2H), 6.99 (d, *J* = 8.7 Hz, 2H), 4.06 (t, *J* = 6.3 Hz, 2H), 2.84 - 2.76 (m, 4H), 2.73 - 2.65 (m, 2H), 1.96 - 1.90 (m, 4H), 1.90 - 1.81 (m, 2H), 1.81 - 1.70 (m, 2H), 1.61 - 1.49 (m, 2H).

¹³C NMR (101 MHz, CDCl₃) δ 190.84, 164.11, 132.02, 129.85, 114.76, 68.03, 56.11, 54.09, 28.80, 27.57, 23.93, 23.40.

### Aldehyde 2e : 4-((5-(piperidin-1-yl)pentyl)oxy)benzaldehyde

Yield: 35%.

¹H NMR (400 MHz, CDCl₃) δ 9.88 (s, 1H), 7.82 (d, *J* = 8.8 Hz, 2H), 6.98 (d, *J* = 8.7 Hz, 2H), 4.04 (t, *J* = 6.5 Hz, 2H), 2.45 - 2.34 (m, 4H), 2.34-2.28 (m, 2H), 1.89 - 1.79 (m, 2H), 1.63 - 1.53 (m, 6H), 1.52 - 1.38 (m, 4H).

¹³C NMR (101 MHz, CDCl₃) δ 190.81, 164.22, 131.99, 129.78, 114.74, 68.24, 59.38, 54.70, 29.01, 26.70, 26.00, 24.49, 24.14.

### Aldehyde 2f : 4-((5-morpholinopentyl)oxy)benzaldehyde

Yield: 26%.

¹H NMR (400 MHz, CDCl₃) δ 9.87 (s, 1H), 7.81 (d, *J* = 8.7 Hz, 2H), 6.97 (d, *J* = 8.7 Hz, 2H), 4.04 (t, *J* = 6.4 Hz, 2H), 3.77- 3.64 (m, 4H), 2.50 - 2.40 (m, 4H), 2.39 - 2.31 (m, 2H), 1.88 - 1.78 (m, 2H), 1.67-1.41 (m, 4H).

¹³C NMR (101 MHz, CDCl₃) δ 190.92, 164.28, 132.11, 129.94, 114.84, 68.29, 67.09, 59.03, 53.91, 29.08, 26.37, 24.04.

### Aldehyde 2g : 4-((6-(pyrrolidin-1-yl)hexyl)oxy)benzaldehyde

Yield: 31%.

¹H NMR (400 MHz, CDCl₃) δ 9.86 (s, 1H), 7.80 (d, *J* = 8.8 Hz, 2H), 6.97 (d, *J* = 8.7 Hz, 2H), 4.02 (t, *J* = 6.5 Hz, 2H), 2.57 - 2.48 (m, 4H), 2.48 - 2.40 (m, 2H), 1.88 - 1.71 (m, 6H), 1.61 - 1.51 (m, 2H), 1.51 - 1.43 (m, 2H), 1.43- 1.34 (m, 2H).

¹³C NMR (101 MHz, CDCl₃) δ 190.92, 164.33, 132.08, 129.85, 114.85, 68.40, 56.59, 54.31, 29.08, 28.91, 27.47, 26.03, 23.49.

### Aldehyde 2h : 4-((6-(piperidin-1-yl)hexyl)oxy)benzaldehyde

Yield: 29%.

¹H NMR (400 MHz, CDCl₃) δ 9.87 (s, 1H), 7.82 (d, *J* = 8.8 Hz, 2H), 6.98 (d, *J* = 8.7 Hz, 2H), 4.03 (t, *J* = 6.5 Hz, 2H), 2.57- 2.42 (m, 3H), 2.42 -2.32 (m, 2H), 1.86 - 1.76 (m, 2H), 1.71 - 1.54 (m, 6H), 1.54-1.42 (m, 4H), 1.42- 1.32 (m, 2H).

¹³C NMR (101 MHz, CDCl₃) δ 190.98, 164.37, 132.13, 129.90, 114.89, 68.45, 59.60, 54.79, 29.13, 27.57, 26.93, 26.07, 26.04, 24.57.

### Aldehyde 2i : 4-((6-morpholinohexyl)oxy)benzaldehyde

Yield: 36%.

¹H NMR (400 MHz, CDCl₃) δ 9.86 (s, 1H), 7.81 (d, *J* = 8.6 Hz, 2H), 6.97 (d, *J* = 8.6 Hz, 2H), 4.02 (t, *J* = 6.4 Hz, 2H), 3.75 - 3.66 (m, 4H), 2.50 -2.37 (m, 4H), 2.37 -2.28 (m, 2H), 1.87- 1.74 (m, 2H), 1.59 - 1.44 (m, 4H), 1.42- 1.32 (m, 2H).

¹³C NMR (101 MHz, CDCl₃) δ 190.89, 164.31, 132.09, 129.90, 114.84, 77.48, 77.16, 76.84, 68.37, 67.06, 59.13, 53.89, 29.11, 27.31, 26.55, 26.03.

### BIGI-BIH-D1: Ethyl 1-(2-(1-benzylpiperidin-4-yl)ethyl)-6-methyl-2-oxo-4-(4-(4-(pyrrolidin-1-yl)butoxy)phenyl)-1,2,3,4-tetrahydropyrimidine-5-carboxylate

Yield: 31%.

¹H NMR (400 MHz, CDCl₃) δ 7.33-7.26 (m, 4H), 7.25 -7.18 (m, 1H), 7.11 (d, *J* = 8.7 Hz, 2H), 6.77 (d, *J* = 8.6 Hz, 2H), 5.90 (s, 1H), 5.26 (d, *J* = 3.0 Hz, 1H), 4.07 (q, *J* = 7.1 Hz, 2H), 4.01 -3.94 (m, 1H), 3.91 (t, *J* = 6.3 Hz, 2H), 3.64 - 3.51 (m, 1H), 3.44 (s, 2H), 2.89 -2.77 (m, 2H), 2.56 - 2.49 (m, 5H), 2.48 (s, 3H), 1.92 - 1.83 (m, 2H), 1.82 - 1.74 (m, 5H), 1.72 - 1.64 (m, 3H), 1.64 - 1.57 (m, 1H), 1.56 - 1.35 (m, 3H), 1.34 - 1.19 (m, 5H), 1.16 (t, *J* = 7.1 Hz, 3H).

¹³C NMR (101 MHz, CDCl₃) δ 166.29, 158.62, 153.55, 148.32, 138.60, 135.82, 129.22, 128.19, 127.50, 126.94, 114.53, 105.01, 67.76, 63.49, 60.17, 56.16, 54.19, 53.73, 53.70, 53.44, 40.57, 36.44, 33.64, 32.33, 32.08, 27.42, 25.49, 23.47, 16.08, 14.24.

### BIGI-BIH-D2: Ethyl 1-(2-(1-benzylpiperidin-4-yl)ethyl)-6-methyl-2-oxo-4-(4-(4-(piperidin-1-yl)butoxy)phenyl)-1,2,3,4-tetrahydropyrimidine-5-carboxylate

Yield: 17%.

¹H NMR (400 MHz, CDCl₃) δ 7.35-7.29 (m, 4H), 7.25 -7.20 (m, 1H), 7.13 (d, *J* = 8.5 Hz, 2H), 6.79 (d, *J* = 8.5 Hz, 2H), 5.33 - 5.22 (m, 2H), 4.08 (q, *J* = 7.1 Hz, 2H), 4.00 - 3.88 (m, 3H), 3.67 - 3.56 (m, 1H), 3.48 (s, 2H), 2.92 - 2.81 (m, 2H), 2.64 - 2.33 (m, 9H), 1.97 - 1.85 (m, 2H), 1.84 - 1.60 (m, 10H), 1.60 - 1.52 (m, 1H), 1.51 - 1.41 (m, 3H), 1.38 - 1.22 (m, 3H), 1.17 (t, *J* = 7.1 Hz, 3H).

¹³C NMR (101 MHz, CDCl₃) δ 166.32, 158.71, 153.42, 148.31, 138.50, 135.87, 129.35, 128.30, 127.63, 127.10, 114.68, 105.07, 67.76, 63.53, 60.30, 58.84, 54.46, 53.79, 53.76, 40.74, 36.50, 33.75, 32.35, 32.11, 27.40, 25.43, 24.16, 23.10, 16.18, 14.32.

### BIGI-BIH-D3: Ethyl 1-(2-(1-benzylpiperidin-4-yl)ethyl)-6-methyl-4-(4-(4-morpholinobutoxy)phenyl)-2-oxo-1,2,3,4-tetrahydropyrimidine-5-carboxylate

Yield: 53%.

¹H NMR (400 MHz, CDCl₃) δ 7.31 - 7.27 (m, 2H), 7.25 - 7.17 (m, 1H), 7.09 (d, *J* = 8.7 Hz, 2H), 6.73 (d, *J* = 8.7 Hz, 2H), 6.59 (bs, 1H), 5.24 (d, *J* = 3.1 Hz, 1H), 4.04 (q, *J* = 7.1 Hz, 2H), 3.96 - 3.83 (m, 3H), 3.71 - 3.60 (m, 4H), 3.57 - 3.46 (m, 3H), 2.96 - 2.81 (m, 2H), 2.44 (s, 3H), 2.43 - 2.38 (m, 4H), 2.38 - 2.31 (m, 2H), 1.95 - 1.86 (m, 2H), 1.78 - 1.69 (m, 2H), 1.69 - 1.53 (m, 4H), 1.52 - 1.34 (m, 2H), 1.34 - 1.20 (m, 2H), 1.19 - 1.15 (m, 1H), 1.13 (t, *J* = 7.1 Hz, 3H).

¹³C NMR (101 MHz, CDCl₃) δ 166.14, 158.37, 153.74, 148.22, 136.67, 135.78, 129.53, 128.20, 127.36, 127.31, 114.33, 105.01, 67.54, 66.71, 62.63, 60.04, 58.42, 53.48, 53.05, 52.92, 40.17, 36.13, 33.04, 31.52, 31.18, 27.11, 22.84, 15.94, 14.14.

### BIGI-BIH-D4: Ethyl 1-(2-(1-benzylpiperidin-4-yl)ethyl)-6-methyl-2-oxo-4-(4-((5-(pyrrolidin-1-yl)pentyl)oxy)phenyl)-1,2,3,4-tetrahydropyrimidine-5-carboxylate

Yield: 44%.

¹H NMR (400 MHz, CDCl₃) δ 7.32 - 7.26 (m, 4H), 7.25 - 7.18 (m, 1H), 7.11 (d, *J* = 8.6 Hz, 2H), 6.77 (d, *J* = 8.6 Hz, 2H), 5.91 (bs, 1H), 5.27 (d, *J* = 2.5 Hz, 1H), 4.07 (q, *J* = 7.1 Hz, 2H), 3.98 - 3.92 (m, 1H), 3.90 (t, *J* = 6.5 Hz, 2H), 3.63 - 3.52 (m, 1H), 3.45 (s, 2H), 2.88 - 2.76 (m, 2H), 2.57- 2.48 (m, 3H), 2.48 (s, 3H), 2.46 - 2.41 (m, 2H), 1.93 - 1.83 (m, 2H), 1.81 - 1.72 (m, 5H), 1.70 - 1.64 (m, 1H), 1.63 - 1.37 (m, 7H), 1.34 - 1.18 (m, 4H), 1.16 (t, *J* = 7.1 Hz, 3H).

¹³C NMR (101 MHz, CDCl₃) δ 166.28, 158.63, 153.57, 148.33, 138.58, 135.78, 129.20, 128.17, 127.48, 126.93, 114.49, 105.01, 67.82, 63.47, 60.15, 56.40, 54.16, 53.71, 53.67, 53.38, 40.53, 36.43, 33.60, 32.32, 32.06, 29.21, 28.54, 24.21, 23.44, 16.07, 14.23.

### BIGI-BIH-D5: Ethyl 1-(2-(1-benzylpiperidin-4-yl)ethyl)-6-methyl-2-oxo-4-(4-((5-(piperidin-1-yl)pentyl)oxy)phenyl)-1,2,3,4-tetrahydropyrimidine-5-carboxylate

Yield: 45%.

¹H NMR (400 MHz, CDCl₃) δ 7.35 - 7.27 (m, 4H), 7.25 - 7.20 (m, 1H), 7.12 (d, *J* = 8.6 Hz, 2H), 6.78 (d, *J* = 8.6 Hz, 2H), 5.51 (d, *J* = 2.8 Hz, 1H), 5.28 (d, *J* = 2.5 Hz, 1H), 4.08 (q, *J* = 7.1 Hz, 2H), 3.99 - 3.93 (m, 1H), 3.91 (t, *J* = 6.3 Hz, 2H), 3.66 - 3.54 (m, 1H), 3.46 (s, 2H), 2.94 - 2.75 (m, 2H), 2.61 - 2.42 (m, 6H), 2.42 - 2.33 (m, 2H), 1.95 - 1.85 (m, 2H), 1.83 - 1.73 (m, 2H), 1.72 - 1.57 (m, 8H), 1.56 - 1.37 (m, 6H), 1.35 - 1.21 (m, 3H), 1.17 (t, *J* = 7.1 Hz, 3H).

¹³C NMR (101 MHz, CDCl₃) δ 166.32, 158.75, 153.49, 148.32, 138.59, 135.79, 129.30, 128.26, 127.58, 127.04, 114.62, 105.07, 67.87, 63.54, 60.27, 59.22, 54.55, 53.78, 53.75, 53.65, 40.68, 36.49, 33.71, 32.37, 32.12, 29.23, 26.29, 25.55, 24.25, 16.15, 14.30.

### BIGI-BIH-D6: Ethyl 1-(2-(1-benzylpiperidin-4-yl)ethyl)-6-methyl-4-(4-((5-morpholinopentyl)oxy)phenyl)-2-oxo-1,2,3,4-tetrahydropyrimidine-5-carboxylate

Yield: 35%.

¹H NMR (400 MHz, CDCl₃) δ 7.37 - 7.27 (m, 8H), 7.25 - 7.21 (m, 1H), 7.12 (d, *J* = 8.6 Hz, 4H), 6.78 (d, *J* = 8.6 Hz, 4H), 5.65 (bs, 2H), 5.28 (d, *J* = 2.9 Hz, 2H), 4.08 (q, *J* = 7.1 Hz, 4H), 3.99 - 3.93 (m, 1H), 3.91 (t, *J* = 6.4 Hz, 5H), 3.75 - 3.65 (m, 8H), 3.59 (ddd, *J* = 14.6, 9.7, 5.1 Hz, 2H), 3.51 (s, 4H), 2.96 - 2.82 (m, 4H), 2.49 (s, 6H), 2.47 - 2.38 (m, 8H), 2.38 - 2.31 (m, 4H), 1.99 - 1.89 (m, 4H), 1.82 - 1.74 (m, 4H), 1.66 (dd, *J* = 32.0, 12.5 Hz, 4H), 1.59 - 1.39 (m, 13H), 1.39 - 1.19 (m, 7H), 1.17 (t, *J=* 7.1 Hz, 6H).

¹³C NMR (101 MHz, CDCl₃) δ 166.29, 158.72, 153.56, 148.28, 135.79, 129.50, 128.34, 127.56, 127.31, 114.58, 105.13, 67.89, 67.04, 63.22, 60.26, 59.05, 53.85, 53.56, 53.51, 53.44, 40.54, 36.37, 33.47, 32.00, 31.71, 29.26, 26.36, 24.11, 16.13, 14.28.

### BIGI-BIH-D7: Ethyl 1-(2-(1-benzylpiperidin-4-yl)ethyl)-6-methyl-2-oxo-4-(4-((6-(pyrrolidin-1-yl)hexyl)oxy)phenyl)-1,2,3,4-tetrahydropyrimidine-5-carboxylate

Yield: 43%.

¹H NMR (400 MHz, CDCl₃) δ 7.31 - 7.27 (m, 2H), 7.23 - 7.17 (m, 1H), 7.10 (d, J = 8.3 Hz, 2H), 6.75 (d, J = 8.2 Hz, 2H), 6.18 (bs, 1H), 5.25 (s, 1H), 4.06 (q, J = 7.1 Hz, 2H), 3.98 - 3.91 (m, 1H), 3.88 (t, J = 6.5 Hz, 2H), 3.62 - 3.50 (m, 1H), 3.43 (s, 2H), 2.87 - 2.76 (m, 2H), 2.53 - 2.45 (m, 7H), 2.44 - 2.36 (m, 2H), 1.91 - 1.80 (m, 2H), 1.79 - 1.69 (m, 6H), 1.69 - 1.55 (m, 2H), 1.55 - 1.47 (m, 3H), 1.47 - 1.39 (m, 3H), 1.39 - 1.30 (m, 2H), 1.30 - 1.18 (m, 3H), 1.15 (t, J = 7.1 Hz, 3H).

¹³C NMR (101 MHz, CDCl₃) δ 166.25, 158.56, 153.62, 148.33, 138.56, 135.76, 129.14, 128.11, 127.41, 126.87, 114.42, 104.97, 67.86, 63.43, 60.08, 56.51, 54.16, 53.67, 53.62, 53.22, 40.41, 36.39, 33.51, 32.28, 32.01 , 29.22, 28.84, 27.47, 26.03, 23.39, 16.01, 14.20.

### BIGI-BIH-D8: Ethyl 1-(2-(1-benzylpiperidin-4-yl)ethyl)-6-methyl-2-oxo-4-(4-((6-(piperidin-1-yl)hexyl)oxy)phenyl)-1,2,3,4-tetrahydropyrimidine-5-carboxylate

Yield: 31%.

¹H NMR (400 MHz, CDCl₃) δ 7.36 - 7.27 (m, 4H), 7.25 - 7.19 (m, 1H), 7.13 (d, *J* = 8.6 Hz, 2H), 6.78 (d, *J* = 8.7 Hz, 2H), 5.42 (d, *J* = 2.1 Hz, 1H), 5.28 (d, *J* = 2.8 Hz, 1H), 4.08 (q, *J* = 7.1 Hz, 2H), 3.99 - 3.93 (m, 1H), 3.90 (t, *J* = 6.4 Hz, 2H), 3.66 - 3.54 (m, 1H), 3.47 (s, 2H), 2.94 - 2.77 (m, 2H), 2.61 - 2.45 (m, 6H), 2.44 - 2.35 (m, 2H), 1.98 - 1.84 (m, 2H), 1.81 - 1.72 (m, 2H), 1.71 - 1.51 (m, 9H), 1.51 -1.40 (m, 5H), 1.39 - 1.20 (m, 5H), 1.17 (t, *J* = 7.1 Hz, 3H).

¹³C NMR (101 MHz, CDCl₃) δ 166.33, 158.81, 153.46, 148.31, 138.59, 135.76, 129.31, 128.27, 127.59, 127.05, 114.65, 105.09, 67.97, 63.55, 60.28, 59.22, 54.48, 53.79, 53.77, 53.70, 40.70, 36.50, 33.72, 32.39, 32.13, 29.28, 27.46, 26.30, 26.07, 25.39, 24.14, 16.16, 14.30.

### BIGI-BIH-D9 : Ethyl 1-(2-(1-benzylpiperidin-4-yl)ethyl)-6-methyl-4-(4-((6-morpholinohexyl)oxy)phenyl)-2-oxo-1,2,3,4-tetrahydropyrimidine-5-carboxylate

Yield: 44%.

¹H NMR (400 MHz, CDCl₃) δ 7.42 - 7.27 (m, 5H), 7.13 (d, *J* = 8.5 Hz, 2H), 6.79 (d, *J* = 8.5 Hz, 2H), 5.42 (bs, 1H), 5.28 (d, *J* = 2.0 Hz, 1H), 4.08 (q, *J* = 7.1 Hz, 2H), 4.01 - 3.94 (m, 1H), 3.91 (t, *J* = 6.4 Hz, 2H), 3.77 - 3.68 (m, 4H), 3.66 - 3.48 (m, 3H), 3.10 - 2.81 (m, 2H), 2.49 (s, 3H), 2.47 - 2.39 (m, 4H), 2.38 - 2.29 (m, 2H), 2.08 - 1.93 (m, 2H), 1.82 - 1.68 (m, 3H), 1.68 - 1.60 (m, 1H), 1.58 - 1.41 (m, 7H), 1.41 - 1.30 (m, 3H), 1.29 - 1.21 (m, 1H), 1.17 (t, *J* = 7.1 Hz, 3H).

¹³C NMR (101 MHz, CDCl₃) δ 166.29, 158.80, 153.53, 148.25, 135.74, 129.75, 128.49, 127.58, 114.65, 105.24, 68.03, 67.04, 63.03, 60.31, 59.17, 53.87, 53.57, 53.44, 40.48, 36.28, 33.28, 31.70, 31.35, 29.32, 27.38, 26.53, 26.13, 16.16, 14.30.

### 2. Biological activities

### H3 receptor binding, cholinesterases inhibition, Gsk3β inhibition and calcium channel blockade

The capacities of the compounds BIGI-BIH D1 to D9 to bind the H3 receptor and to inhibit the activity of the cholinesterases hAChE and hBuChE, have been evaluated. The capacities of these compounds to inhibit Gsk3β and to block calcium channel have also been evaluated and compared to those of reference compounds. The results are collected in Table 1 below.

**Table 1. Results of binding to H3 receptors, cholinesterases inhibition (hAChE and hBuChE), Gsk3β inhibition and calcium channel blockade of the compounds BIGI-BIH D1-D9 and reference compounds**

| | H3R Ki [nM] ± SEM | hAChE IC₅₀ (µM) | hBuChE IC₅₀ (µM) | Gsk3β IC₅₀ [µM] | Calcium Channel % inhibition at 10µM |
|---|---|---|---|---|---|
| BIGI-BIH-D1 | 2.8 ± 0.2 | 0.063±0.001 | 1.715±0.119 | 59,8 ± 5,3 | 5 |
| BIGI-BIH-D2 | 0.6 ± 0.08 | 0.067±0.001 | 0.928±0.032 | 105,9 ± 12,3 | 13 |
| BIGI-BIH-D3 | 12.0 ± 0.5 | 0.123±0.002 | nd | 125,5 ± 19,6 | 23 |
| BIGI-BIH-D4 | 0.5 ± 0.05 | 0.088±0.002 | 1.390±0.066 | 60,5 ± 4,8 | 22 |
| BIGI-BIH-D5 | 0.3 ± 0.07 | 0.067±0.001 | 1.284±0.072 | 26,3 ± 4,1 | 34 |
| BIGI-BIH-D6 | 0.6 ± 0.05 | 0.115±0.002 | nd | 56,6 ± 4,7 | 39 |
| BIGI-BIH-D7 | 0.2 ± 0.04 | 0.089±0.002 | 0.768±0.013 | 30,6 ± 3,9 | 47 |
| BIGI-BIH-D8 | 0.2 ± 0.02 | 0.086±0.002 | 1.253±0.046 | 68,2 ± 0,1 | 60 |
| BIGI-BIH-D9 | 3.0 ± 0.4 | 0.115±0.002 | nd | 38,6 ± 0,5 | 61 |
| (R)(-)-α-METHYLHISTAMINE | 0.2 ± 0.03 | - | - | - | - |
| Pitolisant | 1 to 2,4 | - | - | - | - |
| Thioperamide | 47.0 ± 2.2 | - | - | - | - |
| Donepezil | - | 0.011±0.001 | 6.22±0.77 | - | - |
| SB-415286 | - | - | - | 0.055 | - |
| Nimodipine | - | - | - | - | 38 |

As shown in Table 1 all compounds showed strong affinity against H3R in nanomolar range with Ki ranging from 0.2 to 12nM compared to the thioperamide used as standard.

Concerning the cholinesterases inhibition, all the compounds showed a strong inhibition ranging from 63nM for BIGI-BIH-D1 to 123nM BIGI-BIH-D2.

The blocking effect of the depolarization-induced Ca²⁺ uptake was investigated in SH-SY5Y neuroblastoma cells stimulated with an extracellular concentration of K⁺ 70 mM, which induces the cell Ca²⁺ levels elevation by opening voltage-gated Ca²⁺ channels (VGCC), monitored with the Ca²⁺-sensitive fluorescent dye Fluo-4. Nimodipine was used as positive standard. Percentage of inhibition of the compounds BIGI-BIH D1 to D9 are shown in Table 1. Interestingly, BIGI-BIH-D6 to BIGI-BIH-D9 displayed a Ca²⁺ channel blockade better than Nimodipine.

All Compounds were evaluated for their ability to inhibit GSK-3β, using a luminescence assay, in comparison with SB-415286, a well-known GSK3β inhibitor. As reported in Table 1, the IC₅₀ are ranging from 26.3 for BIGI-BIH-D5 and 105.9 µM for BIGI-BIH-D2. These inhibitions potencies in micromolar range are in line with the activity of other GSK-3β inhibitors based on multipotent inhibitors²⁵. In addition, evidences from in vivo studies indicate that micromolar concentration could be more adequate to produce therapeutic effects in the brain without affecting GSK-3β activity in peripheral tissues²⁶.

### BIBLIOGRAPHY

(1) Querfurth, H. W.; LaFerla, F. M. Alzheimer's Disease. N. Engl. J. Med. 2010, 362 (4), 329-344.
(2) Bartus, R. T.; Dean, R. L.; Beer, B.; Lippa, A. S. The Cholinergic Hypothesis of Geriatric Memory Dysfunction. Science 1982, 217 (4558), 408-414.
(3) Liu, Z.; Zhang, A.; Sun, H.; Han, Y.; Kong, L.; Wang, X. Two Decades of New Drug Discovery and Development for Alzheimer's Disease. RSC Adv. 2017, 7 (10), 6046-6058.
(4) Semagacestat's fall: where next for AD therapies? (accessed Oct 9, 2018).
(5) Kumar, A.; Singh, A.; Ekavali, null. A Review on Alzheimer's Disease Pathophysiology and Its Management: An Update. Pharmacol. Rep. PR 2015, 67 (2), 195-203.
(6) Godyń, J.; Jończyk, J.; Panek, D.; Malawska, B. Therapeutic Strategies for Alzheimer's Disease in Clinical Trials. *Pharmacol. Rep.* **2016,** *68* (1), 127-138.
(7) Li, C.; Götz, J. Tau-Based Therapies in Neurodegeneration: Opportunities and Challenges. Nat. Rev. Drug Discov. 2017, 16 (12), 863-883.
(8) Avila, J.; Pérez, M.; Lim, F.; Gómez-Ramos, A.; Hernández, F.; Lucas, J. J. Tau in Neurodegenerative Diseases: Tau Phosphorylation and Assembly. Neurotox. Res. 2004, 6 (6), 477-482.
(9) Villarroya, M.; Garcia, A. G.; Marco-Contelles, J.; López, M. G. An Update on the Pharmacology of Galantamine. Expert Opin. Investig. Drugs 2007, 16 (12), 1987-1998.
(10) Godyń, J.; Jończyk, J.; Panek, D.; Malawska, B. Therapeutic Strategies for Alzheimer's Disease in Clinical Trials. *Pharmacol. Rep.* **2016**, *68* (1), 127-138.
(11) Chen, C.-H.; Zhou, W.; Liu, S.; Deng, Y.; Cai, F.; Tone, M.; Tone, Y.; Tong, Y.; Song, W. Increased NF-KB Signalling up-Regulates BACE1 Expression and Its Therapeutic Potential in Alzheimer's Disease. Int. J. Neuropsychopharmacol. 2012, 15 (1), 77-90.
(12) Cho, H. J.; Jin, S. M.; Youn, H. D.; Huh, K.; Mook-Jung, I. Disrupted Intracellular Calcium Regulates BACE1 Gene Expression via Nuclear Factor of Activated T Cells 1 (NFAT 1) Signaling. Aging Cell 7 (2), 137-147.
(13) Hayley, M.; Perspicace, S.; Schulthess, T.; Seelig, J. Calcium Enhances the Proteolytic Activity of BACE1: An in Vitro Biophysical and Biochemical Characterization of the BACE1-Calcium Interaction. Biochim. Biophys. Acta 2009, 1788 (9), 1933-1938.
(14) Oulès, B.; Del Prete, D.; Greco, B.; Zhang, X.; Lauritzen, I.; Sevalle, J.; Moreno, S.; Paterlini-Bréchot, P.; Trebak, M.; Checler, F.; et al. Ryanodine Receptor Blockade Reduces Amyloid-β Load and Memory Impairments in Tg2576 Mouse Model of Alzheimer Disease. J. Neurosci. Off. J. Soc. Neurosci. 2012, 32 (34), 11820-11834.
(15) Small, D. H.; Gasperini, R.; Vincent, A. J.; Hung, A. C.; Foa, L. The Role of Aβ-Induced Calcium Dysregulation in the Pathogenesis of Alzheimer's Disease. J. Alzheimers Dis. 2009, 16 (2), 225-233.
(16) Avila, J.; Pérez, M.; Lim, F.; Gómez-Ramos, A.; Hernández, F.; Lucas, J. J. Tau in Neurodegenerative Diseases: Tau Phosphorylation and Assembly. Neurotox. Res. 2004, 6 (6), 477-482.
(17) Chakroborty, S.; Stutzmann, G. E. Calcium Channelopathies and Alzheimer's Disease: Insight into Therapeutic Success and Failures. Eur. J. Pharmacol. 2014, 739, 83-95.
(18) Baki, A.; Bielik, A.; Molnár, L.; Szendrei, G.; Keserü, G. M. A High Throughput Luminescent Assay for Glycogen Synthase Kinase-3beta Inhibitors. Assay Drug Dev. Technol. 2007, 5 (1), 75-83.
(19) Coghlan, M. P.; Culbert, A. A.; Cross, D. A.; Corcoran, S. L.; Yates, J. W.; Pearce, N. J.; Rausch, O. L.; Murphy, G. J.; Carter, P. S.; Roxbee Cox, L.; et al. Selective Small Molecule Inhibitors of Glycogen Synthase Kinase-3 Modulate Glycogen Metabolism and Gene Transcription. Chem. Biol. 2000, 7 (10), 793-803.
(20) Gandini, A.; Bartolini, M.; Tedesco, D.; Martinez-Gonzalez, L.; Roca, C.; Campillo, N. E.; Zaldivar-Diez, J.; Perez, C.; Zuccheri, G.; Miti, A.; et al. Tau-Centric Multitarget Approach for Alzheimer's Disease: Development of First-in-Class Dual Glycogen Synthase Kinase 3β and Tau-Aggregation Inhibitors. J. Med. Chem. 2018, 61 (17), 7640-7656.
(21) Walter, M.; Stark, H. Histamine Receptor Subtypes: A Century of Rational Drug Design. Front. Biosci. Sch. Ed. 2012, 4, 461-488.
(22) Ellenbroek, B. A.; Ghiabi, B. The Other Side of the Histamine H3 Receptor. Trends Neurosci. 2014, 37 (4), 191-199.
(23) Bautista-Aguilera, Ó. M.; Hagenow, S.; Palomino-Antolin, A.; Farré-Alins, V.; Ismaili, L.; Joffrin, P.-L.; Jimeno, M. L.; Soukup, O.; Jano 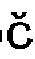 ková, J.; Kalinowsky, L.; et al. Multitarget-Directed Ligands Combining Cholinesterase and Monoamine Oxidase Inhibition with Histamine H3 R Antagonism for Neurodegenerative Diseases. *Angew. Chem. Int. Ed Engl.* **2017,** *56* (41), 12765-12769.
(24) Bautista-Aguilera, Ó. M.; Budni, J.; Mina, F.; Medeiros, E. B.; Deuther-Conrad, W.; Entrena, J. M.; Moraleda, I.; Iriepa, I.; López-Muñoz, F.; Marco-Contelles, J. Contilisant, a Tetratarget Small Molecule for Alzheimer's Disease Therapy Combining Cholinesterase, Monoamine Oxidase Inhibition, and H3R Antagonism with S1R Agonism Profile. J. Med. Chem. 2018, 61 (15), 6937-6943.
(25) Prati, F.; De Simone, A.; Bisignano, P.; Armirotti, A.; Summa, M.; Pizzirani, D.; Scarpelli, R.; Perez, D. I.; Andrisano, V.; Perez-Castillo, A.; Monti, B.; Massenzio, F.; Polito, L.; Racchi, M.; Favia, A. D.; Bottegoni, G.; Martinez, A.; Bolognesi, M. L.; Cavalli, A. Multitarget Drug Discovery for Alzheimer's Disease: Triazinones as BACE-1 and GSK-3β Inhibitors. Angewandte Chemie International Edition 2015, 54 (5), 1578-1582.
(26) Avrahami, L.; Licht-Murava, A.; Eisenstein, M.; Eldar-Finkelman, H. GSK-3 Inhibition: Achieving Moderate Efficacy with High Selectivity. Biochimica et Biophysica Acta (BBA) - Proteins and Proteomics 2013, 1834 (7), 1410-1414.

## Claims

1. A compound of formula (I): 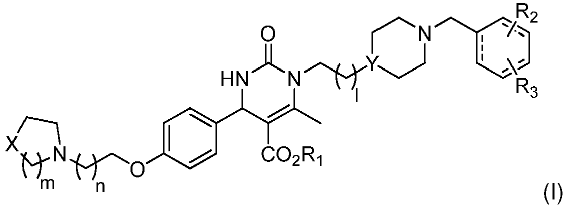 including any stereochemically isomeric form thereof, wherein :
Y is CH or N,
I is 0 or 1,
R₁ is a substituted or unsubstituted C₁-C₆ alkyl or a substituted or unsubstituted aryl,
R₂ and R₃ are independently of each other, are H, halogen, substituted or unsubstituted C₁-C₆ alkyl, cycloalkyl, substituted or unsubstituted aryl, NO₂, OH, CN, acyl, alkoxy, alkoxycarbonyl, SH,
X is O or CH₂,
m is 1 or 2,
n is 3, 4 or 5,
or a pharmaceutically acceptable salt thereof.

2. The compound according to claim 1, wherein R₂ and R₃ are H.

3. The compound according to claim 1 or 2, wherein R₁ is an alkyl, preferably an ethyl.

4. The compound according to any one of claims 1 to 3, wherein Y is CH and wherein R₂ and R₃ are H.

5. The compound according to any one of claims 1 to 4, wherein Y is CH, wherein R₂ and R₃ are H and wherein R₁ is an ethyl.

6. The compound of formula (II) according to any one of claims 1 to 5, wherein Y is CH, R₂ and R₃ are H, R₁ is an ethyl and I is 1.

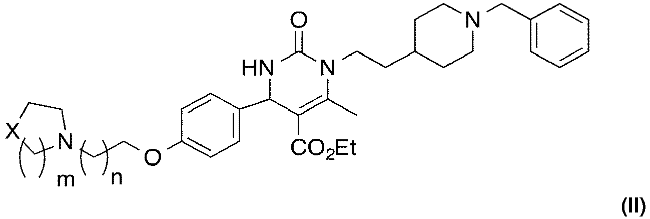

7. The compound according to any one of claims 1 to 6, wherein X is CH₂.

8. The compound according to any one of claims 1 to 6, wherein X is O..

9. The compound according to any one of claims 1 to 8, wherein m is 1.

10. The compound according to any one of claims 1 to 8, wherein m is 2.

11. The compound according to any one of claims 1 to 6, wherein said compound is chosen in the group consisting of:

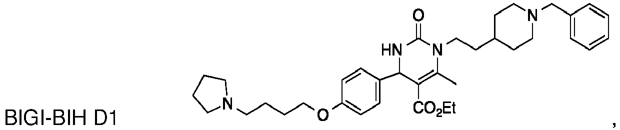

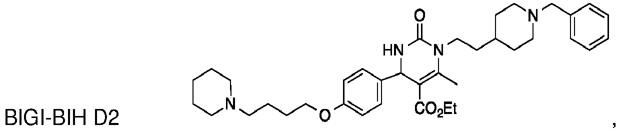

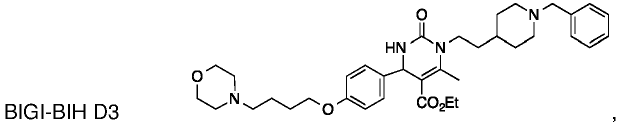

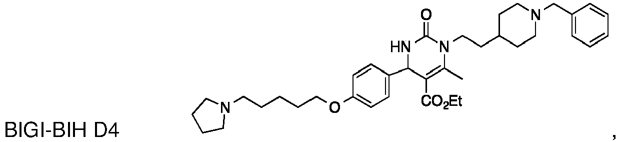

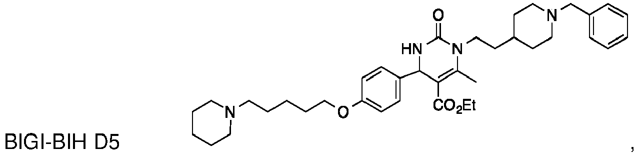

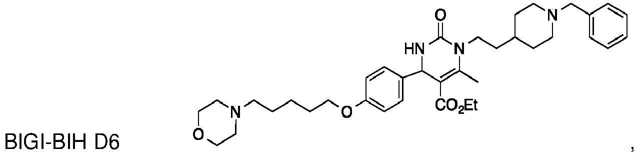

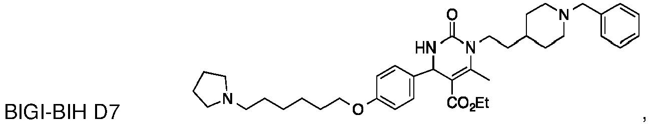

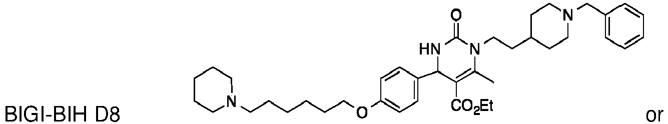

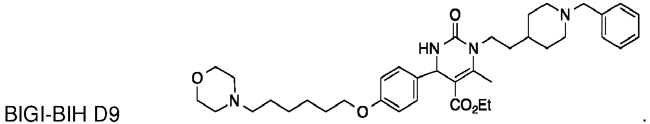

12. A pharmaceutical composition comprising a compound according to any of claims 1 to 11, or a pharmaceutically acceptable salt thereof, and a pharmaceutically acceptable carrier.

13. A compound according to any of claims 1 to 11 for use as a medicament.

14. A compound according to any of claims 1 to 11, for use in the treatment of a central nervous system disease, narcolepsy, a neurodegenerative disease or sleep disorder.

15. A compound for use according to claim 14, wherein said neurodegenerative disease is selected in the group consisting of: Alzheimer's disease, Parkinson's disease, amyotrophic lateral sclerosis.

## Patentansprüche

1. Verbindung der Formel (I): 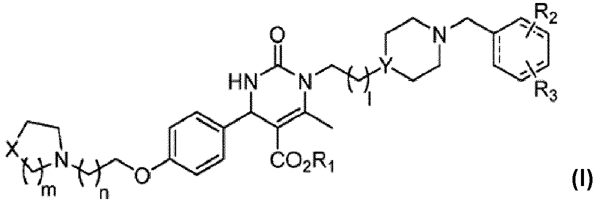 einschließlich jeder stereochemisch isomeren Form davon, wobei:
Y CH oder N ist,
I 0 oder 1 ist,
R₁ ein substituiertes oder unsubstituiertes C₁-C₆-Alkyl oder ein substituiertes oder unsubstituiertes Aryl ist,
R₂ und R₃ unabhängig voneinander H, Halogen, substituiertes oder unsubstituiertes C₁-C₆-Alkyl, Cycloalkyl, substituiertes oder unsubstituiertes Aryl, NO₂, OH, CN, Acyl, Alkoxy, Alkoxycarbonyl, SH sind,
X O oder CH₂ ist,
m 1 oder 2 ist,
n 3, 4 oder 5 ist,
oder ein pharmazeutisch akzeptables Salz davon.

2. Verbindung nach Anspruch 1, wobei R₂ und R₃ H sind.

3. Verbindung nach Anspruch 1 oder 2, wobei R₁ ein Alkyl, vorzugsweise ein Ethyl ist.

4. Verbindung nach einem der Ansprüche 1 bis 3, wobei Y CH ist und wobei R₂ und R₃ H sind.

5. Verbindung nach einem der Ansprüche 1 bis 4, wobei Y CH ist, wobei R₂ und R₃ H sind und wobei R₁ ein Ethyl ist.

6. Verbindung der Formel (II) nach einem der Ansprüche 1 bis 5, wobei Y CH ist, R₂ und R₃ H sind, R₁ ein Ethyl ist und I 1 ist.

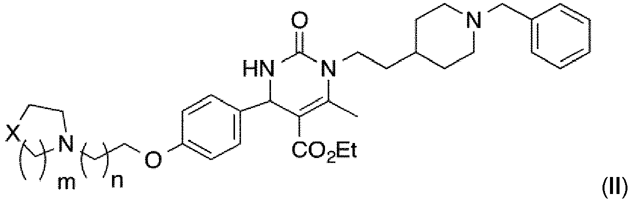

7. Verbindung nach einem der Ansprüche 1 bis 6, wobei X CH₂ ist.

8. Verbindung nach einem der Ansprüche 1 bis 6, wobei X O ist.

9. Verbindung nach einem der Ansprüche 1 bis 8, wobei m 1 ist.

10. Verbindung nach einem der Ansprüche 1 bis 8, wobei m 2 ist.

11. Verbindung nach einem der Ansprüche 1 bis 6, wobei die Verbindung ausgewählt ist aus der Gruppe bestehend aus: 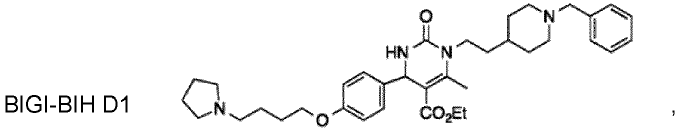 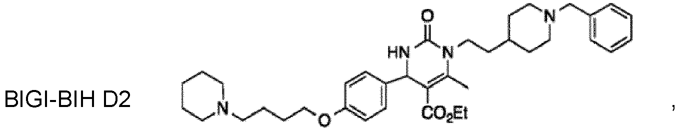 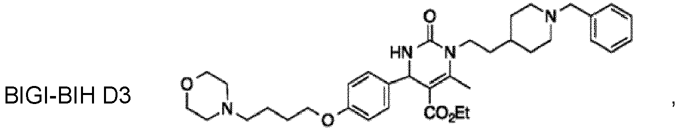 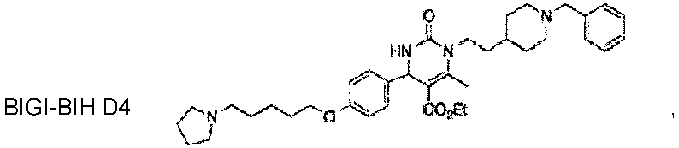 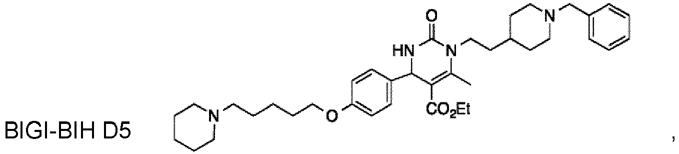 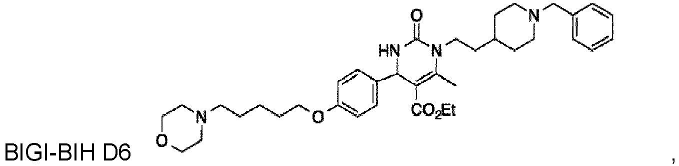 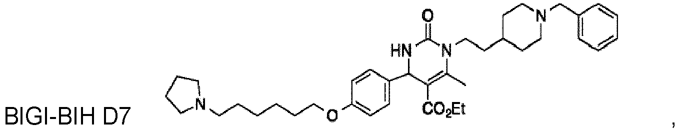 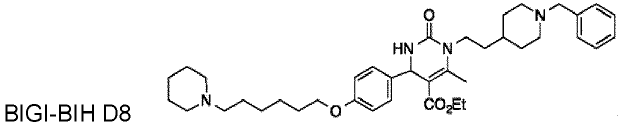 or

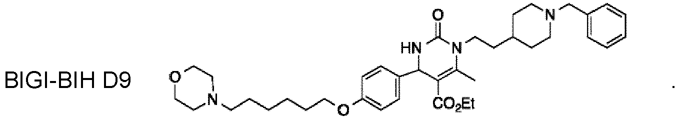

12. Pharmazeutische Zusammensetzung, umfassend eine Verbindung nach einem der Ansprüche 1 bis 11 oder ein pharmazeutisch akzeptables Salz davon und einen pharmazeutisch akzeptablen Träger.

13. Eine Verbindung nach einem der Ansprüche 1 bis 11 zur Verwendung als Medikament.

14. Verbindung nach einem der Ansprüche 1 bis 11 zur Verwendung bei der Behandlung einer Erkrankung des zentralen Nervensystems, Narkolepsie, einer neurodegenerativen Erkrankung oder einer Schlafstörung.

15. Verbindung zur Verwendung nach Anspruch 14, wobei besagte neurodegenerative Krankheit aus der Gruppe ausgewählt ist, bestehend aus: Alzheimer-Krankheit, Parkinson-Krankheit, amyotrophe Lateralsklerose.

## Revendications

1. Composé de formule (I) : 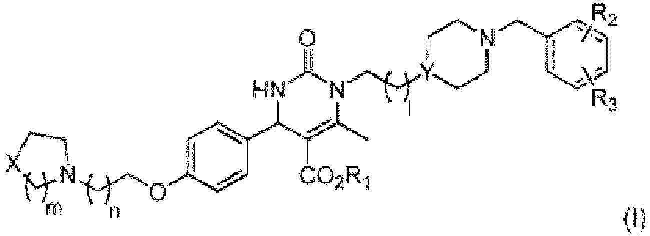 incluant toute les formes stéréochimiquement isomères de celui-ci, dans lequel :
Y est CH ou N,
1 est 0 ou 1,
R₁ est un alkyle en C₁-C₆ substitué ou non substitué ou un aryle substitué ou non substitué,
R₂ et R₃, indépendamment l'un de l'autre, sont H, un halogène, un alkyle en C₁-C₆ substitué ou non substitué, un cycloalkyle, un aryle substitué ou non substitué, NO₂, OH, CN, un acyle, un alcoxy, un alcoxycarbonyle, SH,
X est O ou CH₂,
m est 1 ou 2,
n est 3, 4 ou 5,
ou un sel pharmaceutiquement acceptable de celui-ci.

2. Composé selon la revendication 1, dans lequel R₂ et R₃ sont H.

3. Composé selon la revendication 1 ou 2, dans lequel R₁ est un alkyle, de préférence un éthyle.

4. Composé selon l'une quelconque des revendications 1 à 3, dans lequel Y est CH et dans lequel R₂ et R₃ sont H.

5. Composé selon l'une quelconque des revendications 1 à 4, dans lequel Y est CH, dans lequel R₂ et R₃ sont H et dans lequel R₁ est un éthyle.

6. Composé de formule (II) selon l'une quelconque des revendications 1 à 5, dans lequel Y est CH, R₂ et R₃ sont H, R₁ est un éthyle et 1 est 1.

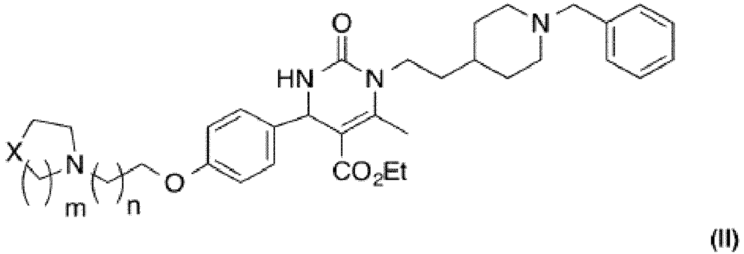

7. Composé selon l'une quelconque des revendications 1 à 6, dans lequel X est CH₂.

8. Composé selon l'une quelconque des revendications 1 à 6, dans lequel X est O.

9. Composé selon l'une quelconque des revendications 1 à 8, dans lequel m est 1.

10. Composé selon l'une quelconque des revendications 1 à 8, dans lequel m est 2.

11. Composé selon l'une quelconque des revendications 1 à 6, dans lequel ledit composé est choisi dans le groupe constitué de :

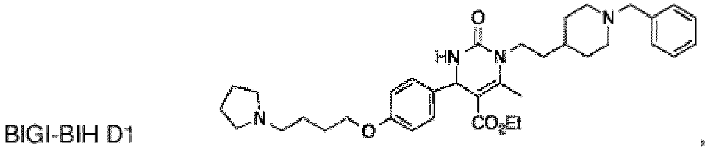

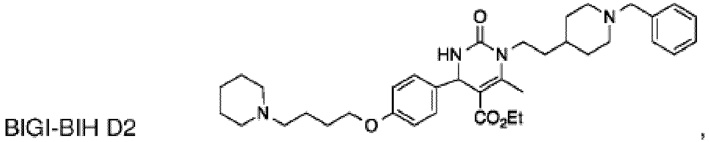

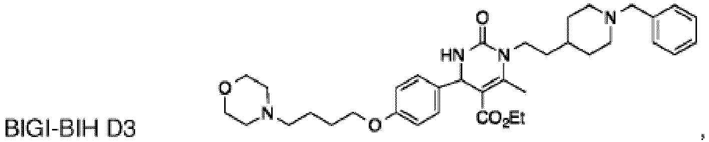

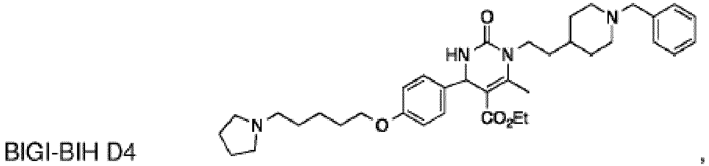

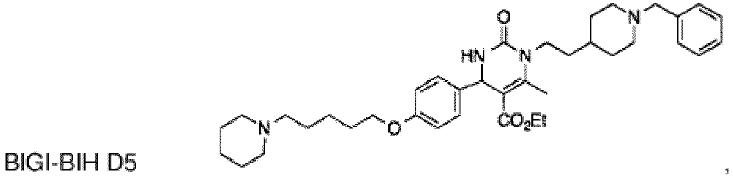

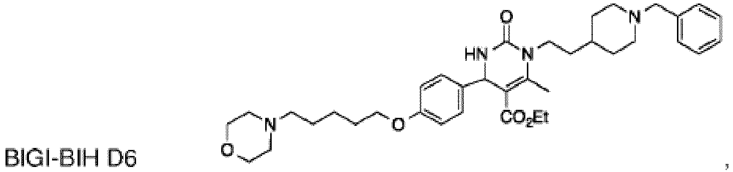

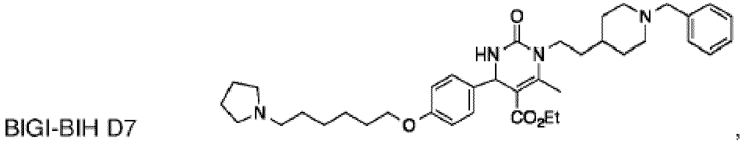

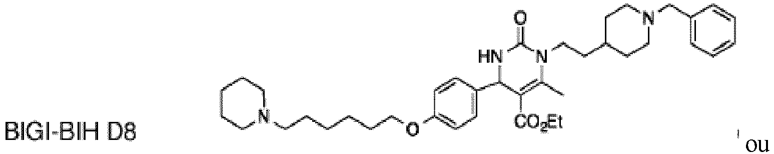

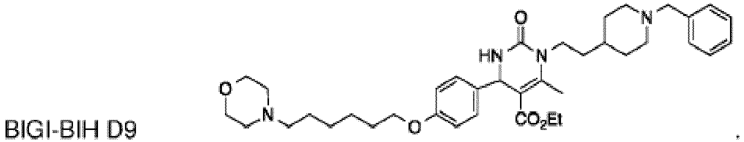

12. Composition pharmaceutique comprenant un composé selon l'une quelconque des revendications 1 à 11, ou un sel pharmaceutiquement acceptable de celui-ci, et un support pharmaceutiquement acceptable.

13. Composé selon l'une quelconque des revendications 1 à 11, pour une utilisation comme médicament.

14. Composé selon l'une quelconque des revendications 1 à 11, pour une utilisation dans le traitement d'une maladie du système nerveux central, de la narcolepsie, d'une maladie neurodégénérative ou d'un trouble du sommeil.

15. Composé pour une utilisation selon la revendication 14, dans lequel ladite maladie neurodégénérative est sélectionnée dans le groupe constitué de : la maladie d'Alzheimer, la maladie de Parkinson, la sclérose latérale amyotrophique.
